# EUROPEAN PATENT SPECIFICATION

(11) **EP 2 141 009 B1**
(45) Date of publication and mention of the grant of the patent: **26.10.2011**
(21) Application number: 08159536.5
(22) Date of filing: 02.07.2008
(51) Int. Cl.: B32B 5/24, B32B 5/18, B32B 7/02, B32B 27/08, B32B 27/12, B32B 27/02, B32B 27/36, B32B 27/40, B32B 27/34, B32B 1/00, A61B 6/04

(54) **Material, cushion and support for immobilising a subject for medical treatment**
Material, Kissen und Unterlage zur Ruhigstellung einer Person für medizinische Behandlungen
Matériau, coussin et support pour immobiliser un sujet en vue d'un traitement médical

(43) Date of publication of application: 06.01.2010
(73) Proprietor: T Tape Company BV, 4645 EX Putte (NL)
(72) Inventor: Nieberding, Reginald, 4645 EX Putte (NL)
(74) Representative: Brants, Johan P.E.

(56) References cited:
- EP-A- 1 174 088
- EP-A- 1 537 831
- DE-A1- 2 216 366
- DE-C1- 19 824 691
- US-A- 5 566 681

## Description

The present invention concems a material and device for supporting and immobilising a portion of a subject for medical treatment. In particular it concems a device for immobilising the head and neck, or other parts of the body of a subject for radiotherapy treatment.

### BACKGROUND TO THE INVENTION

Patients being treated by radiotherapy for a tumorous growth are typically required to receive radiation doses at regular intervals, whereby each dose is precisely directed to the same location of the body. This necessitates securing devices that are adapted to the shape of the body portion being treated, which helps accurately and quickly to align the radiation source with the location of the tumour.

Commonly, tables or chairs at the radiation source, *e.g.*, linear accelerator, CT machine, etc., support the patient in a prone or supine position while the relevant portion of the patient's body is held in a fixed or immobilised condition. The aforementioned securing device immobilises the subject by mounting the body part on a patient support table where treatment is delivered.

For example, the treatment of cancer to the head, neck or throat entails that a rigid mask molded to conform to the contours of the patient's face, is placed over the subject's face that immobilises the head, which mask precisely locates onto a patient support table whose position relative to the source is calibrated. The head of the subject may be placed accurately and repeatedly, owing to the mask which immobilises the head relative to the source.

An example of a head immobilization assembly for positioning a patient for radiotherapy treatment is given in EP 1 537 831.

Known the art is a device for immobilising the head of a subject that comprises a mask of thermoplastic sheet material molded to fit the face, and a second mask molded for fit the back of the head. The person who is to undergo radiotherapy is first placed In a prone position and a first thermoplastic material sheet is placed over the back of his head and neck. The subjects head is pushed onto a cushion and the thermoplastic material sheet is allowed to conform to the contours of the back portion of his head and neck. Once the first thermoplastic sheet has been conformed and has hardened, the subject is then placed in a supine position and the first sheet is inverted such that his head neck is supported by it. A second thermoplastic sheet or mask is then used to conform to the contours of the subject's face. After the face mask has hardened the two rigid masks can then be used as a unit to hold and immobilize the subject's head and neck for radiation therapy or any other purpose.

The problems in art concern the degree of physiological and psychological discomfort experienced by the patient during the molding process. Subjects report feeling of claustrophobia, in addition to undesirable reactions to the materials used to prepare the masks. There is a need in the art for a thermoplastic material that provides more comfort to the subject, and for a system that expedites the molding process. The present invention provides a new material for preparing a mask that provides more comfort without sacrificing the requisite immobilisation qualities, and also a device that serves both to expedite molding and secure the subject to the treatment table. It further provides an inflatable cushion made from the new material that allows a mold to be taken more reliably while maximizing comfort to the subject. The cushion is particularly effective at avoiding false impressions that arise when deep impressions are taken and the thermoplastic material contacts and becomes limited by the dimensions of the molding device, thereby giving an incorrect impression.

### SUMMARY OF THE INVENTION

One embodiment of the invention is a flexible thermoplastic sheet material (100), suitable for molding to and immobilising a part of a subject during medical treatment, comprising:
- a core layer (10) having an upper surface (1) and lower surface (2), that is a thermoplastic composition comprising polycaprolactone and polyurethane,
- a first outer layer (5) disposed over the upper surface of the core layer, comprising a material formed from a yarn comprising polyamide and elastane,
- a second outer layer (15) disposed over the lower surface of the core layer comprising open cell foam,
which layers bonded so as to form a single sheet.

Another embodiment of the invention is a flexible thermoplastic sheet material (100) as described above, wherein the core layer (10) comprises 20 % to 40 %, polyurethane, and 60 % to 80 % (w/w) polycaprolactone.

Another embodiment of the invention is a flexible thermoplastic sheet material (100) as described above, wherein the core layer (10) further comprises between 1 to 40 % (w/w) of non-metallic, heat-accumulating microspheres.

Another embodiment of the invention is a flexible thermoplastic sheet material (100) as described above, wherein the yarn of the first outer layer (5) comprises between 80 % to 95 % polyamide, and between 5 % and 15 % elastane.

Another embodiment of the invention is a flexible thermoplastic sheet material (100) as described above, wherein the fabric weight of the first outer layer (5) is between 210 g/m² and 230 g_{/}m².

Another embodiment of the invention is a flexible thermoplastic sheet material (100) as described above, wherein the thickness of the first outer layer (5) is between 0.05 and 1.5 mm.

Another embodiment of the invention is a flexible thermoplastic sheet material (100) as described above, wherein the second outer layer (15) is made from polyurethane, polyester polyurethane or polyether open-cell foam.

Another embodiment of the invention is a flexible thermoplastic sheet material (100) as described above, further comprising an intervening layer (12, 12') disposed between the core layer (10) and the first outer layer (5), and/or disposed between the core layer (10) and the second outer layer (15), made from the same material as the core (10) and with a higher polycaprolactone content.

Another embodiment of the invention is a flexible thermoplastic sheet material (100) as described above, having a maximum total thickness of 1.5 to 1.7 mm.

Another embodiment of the invention is an inflatable cushion (300) dimensioned to receive a body portion comprising
- a first flexible sheet (110) made at least partially from a thermoplastic sheet (100) as described above
- a second flexible sheet (115), optionally made at least partially from a thermoplastic sheet as described above,
which sheets are sealed together so as form a lumen (117) that can receive and retain inflation medium, and
- an inflation nozzle (140) for the passage of inflation medium to the lumen (117).

Another embodiment of the invention is an inflatable cushion (300) as describe above, further comprising one or more side pleats (130) between the first (110) and second (115) flexible sheets.

Another embodiment of the invention is an inflatable cushion (300) as describe above, further comprising an attachment means (120, 125, 143, 145) for attachment of the cushion to a molding frame (300).

Another embodiment of the invention is an inflatable cushion (300) as describe above, formed from a single piece of thermoplastic sheet (100) as described above, folded and sealed so as form the lumen (117).

Another embodiment of the invention is an inflatable cushion (300) comprising
- a first flexible sheet (110) made at least partially from a sheet (100) as described above,
- a second flexible sheet (115), optionally made at least partially from a sheet as described above,
which sheets are sealed together so as form a lumen (117) that can receive and retain inflation medium, and
- an inflation nozzle (140) for the passage of inflation medium to the lumen (117).

Another embodiment of the invention is an inflatable cushion (300) as described above, further comprising one or more side pleats (130) between the first (110) and second (115) flexible sheets.

Another embodiment of the invention is an inflatable cushion (300) as described above, formed from a single piece of thermoplastic sheet (100) as described above, folded and sealed so as form the lumen (117).

### FIGURE LEGENDS

**FIG.1** depicts a schematic drawing of a cross section through a sheet of thermoplastic material according to the invention.
**FIG. 2** depicts a schematic drawing of a cross section through a sheet of thermoplastic material according to the invention, further comprising intervening layers.
**FIG. 3A** depicts a plan view of a sheet of the invention after molding to the back of a head and neck of a subject, whereby contour lines show the relief of the impression.
**FIG. 3B** depicts a cross section of the sheet of **FIG. 3A**
**FIG. 4** shows a plan view of sheet of the invention, disposed with attachment means.
**FIG. 5** shows perspective view of an inflatable cushion of the invention disposed with a nozzle at a folded end.
**FIG. 6** shows perspective view of an inflatable cushion of the invention disposed with a nozzle at a side pleat.
**FIG. 7A** shows perspective view of a molding frame of the invention.
**FIG. 7B** shows a transverse (A-A') cross section through a longitudinal support of **FIG. 7A****.**
**FIG. 8** shows perspective view of a molding frame of the invention disposed with a sheet of the invention.
**FIG. 9A** shows perspective view of a molding frame of the invention disposed with an inflatable cushion of the invention.
**FIG. 9B** shows a transverse cross section through a longitudinal support of **FIG. 8A.**
**FIG. 10** shows perspective view of a molding frame of the invention wherein the depth of the opening at the second end **68** is greater than that at the first end **66.**
**FIG. 11A** shows a perspective view of a cushion of the invention attached to a pair of buffering structures.
**FIG. 11B** shows a transverse (B-B') cross section through the buffering structures and cushion of **FIG. 11A****.**

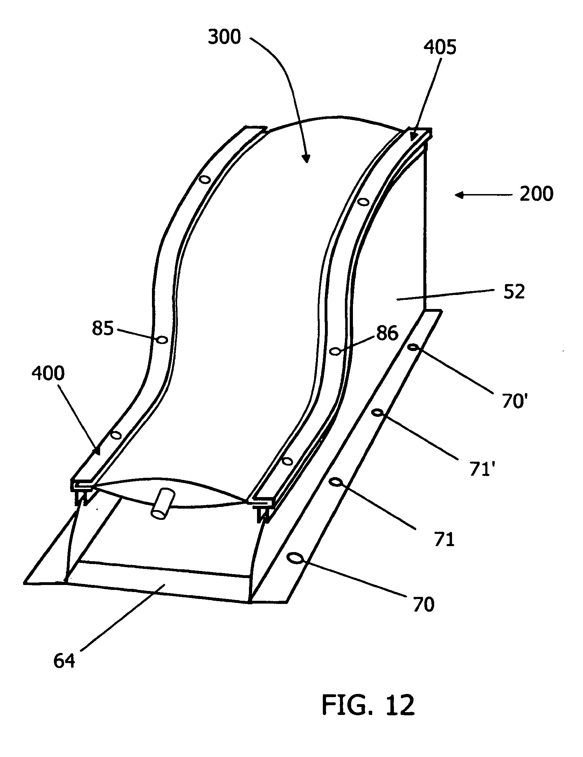

**FIG. 12** shows perspective view of a molding frame of the invention attached by a buffering structure to a cushion of the invention.
**FIG. 13** shows a transverse cross section through the supports and inflated cushion and of **FIG. 9****,** and the body part to be applied.
**FIG. 14** shows a transverse cross section through the supports and inflated cushion and of **FIG. 9** after the application of the body part.
**FIG. 15** shows a transverse cross section through the buffering structures and cushion of the invention, depicting a variation of the buffering structures.
**FIG. 16** shows a transverse cross section through the buffering structures and cushion of the invention, depicting a further variation of the buffering structures.
**FIG. 17A** shows a plan view of a bed frame disposed with rails to receive a cushion of the invention.
**FIG. 17B** shows a side view along the first end of a bed frame of **FIG. 17A** disposed with rails to receive a cushion of the invention.
**FIG. 18A** shows a plan view of a frame according to the invention, **FIG. 18B** shows the side elevation and **FIG. 18C** shows a front elevation.

### DETAILED DESCRIPTION OF THE INVENTION

The present invention provides a thermoplastic sheet material **100,** suitable for molding to and immobilising a part of a subject during medical treatment, comprising:
- a core layer **10** having an first (*e.g.* upper) surface **1** and a second (*e.g.* lower) surface **2,** that is a thermoplastic composition comprising polycaprolactone and polyurethane ,
- a first outer layer **5** disposed over the upper surface of the core layer, formed from a yam comprising polyamide and elastane, and
- a second outer layer **15** disposed over the lower surface of the core layer comprising open cell foam,
which layers bonded so as to form a single sheet.

The sheet exhibits excellent deformability properties, conforming to the shape of the body part without the need to apply excessive pressure. The first layer 5 provides a comfortable wearing against the skin, and prevents adhesion to the skin and/or hair by the core layer 10. The second layer has been found to reduce hardening time and prevents the core 10 from adhering to itself or other articles.

The invention also provides an inflatable cushion 300 made at least partly from the

A device is also described herein for molding a sheet **100** prior to medical treatment, and/or for immobilising a subject during medical treatment, especially radiotherapy for instance to the head and neck, comprising a frame **200** that Is configured to immobilise and support a sheet of material **100** or cushion **300** over an opening, which opening is dimensioned to receive the impression of a body part.

### Material

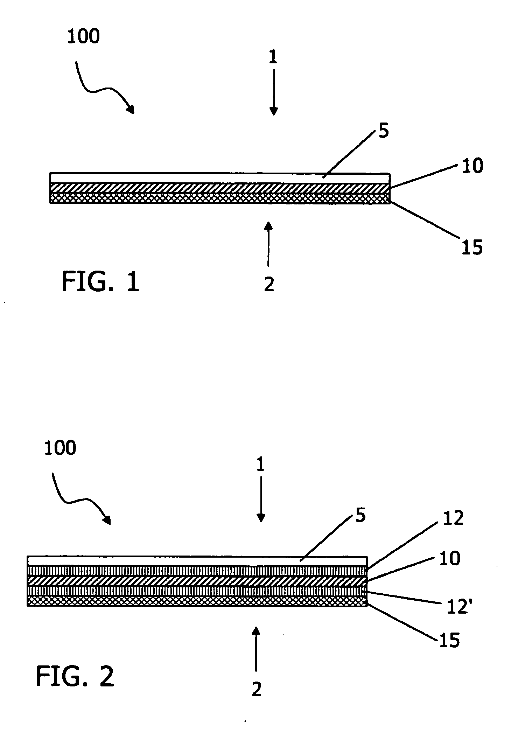

The present invention provides a thermoplastic sheet material deformable under the application of heat to conform to the contours of a portion (*e.g.* head and neck) of a subject, which after cooling down retains the deformed shape and becomes rigid or semi-rigid. With reference to **FIG. 1** the sheet material **100** comprises at least three separate layers - a core layer **10** having an first **1** (skin facing) surface and lower surface **2,** a first outer layer **5** disposed over the first **1** surface of the core layer and a second outer layer **15** disposed over the second **2** surface of the core layer - which layers are bonded so as to form a single sheet **100.** The overall thickness of the sheet **100** is 1.5 mm, 1.6 mm, 1.7 mm, 1.8 mm, 1.9 mm, 2.0 mm, 2.2 mm, 2.4 mm, 2.5 mm, 2.6 mm, 2.7 mm, 2.8 mm, 2.9 mm, 3.0 mm, 3.2 mm, 3.4 mm, 3.5 mm, 3.6 mm, 3.7 mm, 3.8 mm, 3.9 mm, 4.0 mm depending on the which part of the body needs to be immobilized.

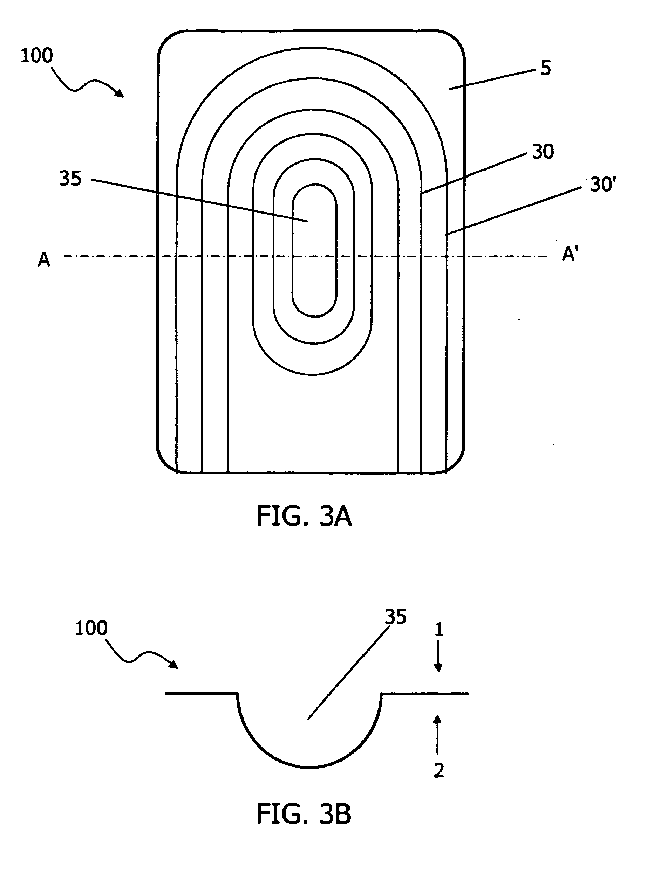

The sheet **100** is distortable at temperatures of between **50** and **70** deg Celsius, depending on the polycaprolactone content. Typically the sheet is brought into the malleable condition by heating in a water bath set around 5 degrees Celsius above the melting temperature, most preferable at 65 deg Celsius. An impression of the body part is taken, for example of the back of the head, by placing the first surface **1** *i.e*. the first outer layer **5** in contact with the skin and/or hair, and applying pressure to the sheet **100.** After molding, the sheet **100** is allowed to cool, hardening in the process. A resulting mask is shown in **FIGs. 3A** and **3B****.** In **FIG. 3A****,** the impression of the body part is illustrated as a series of contour lines **30, 30'** which indicate increasing depths from the edges of the sheet **30, 30'** towards a central deepest region 35 A cross-section along line **A-A'** is shown in **FIG. 3B****.**

The sheet **100** exhibits excellent deformability properties, conforming to the shape of the body part without the need to apply excessive pressure. The first layer **5** provides a comfortable wearing against the skin, and prevents adhesion to the skin and/or hair by the core layer **10.** It also insulates the heat emitted by the heated core **10** layer from the skin of the subject. The second layer **15** prevents the core **10** from adhering to itself or other articles, and in addition reduces the hardening time. Further, the multi-layer sheets can be rolled in the deformed condition without damage to the integrity of the structure. The sheet used for the support of the head, being considerably thinner than conventional masks is semi-rigid, has a soft touch feeling, and, therefore, provides a high degree of comfort in both molding and wearing.

### Core layer

The core layer **10** comprises a thermoplastic composition containing polycaprolactone and polyurethane and having a thickness between 1 mm, 1.5 mm, 2.0 mm, 2.5 mm, 3 mm or a value in the range between any two of the aforementioned values, preferably between 1 mm 3 mm.

The polyurethane may present in an amount of 10%, 20 %, 30 %, 40 % or 50% (w/w), or a value in the range between any two of the aforementioned values, preferably 20 to 40 %, most preferably 30%. The polycaprolactone may be present in an amount of 60 %, 70 %, 80 % or 90% (w/w) or a value in the range between any two of the aforementioned values, preferably 60 to 80 % (w/w). most preferably 70%. Typically, there will be more polycaprolactone than polyurethane which polycaprolactone lowers the temperature at which the sheet deforms. The ratio of polycaprolactone:polyurethane is (w:w) may be 5:1, 4:1. 3:2. 3:1,, 2.3:1, 2:1 preferably 2.3:1.

The molecular weight of the polyurethane may be equal to or less than 10 000, 20 000, 30 000, 40 000, 50 000, 60 000, 70 000, 80 000, 90 000, 100 000, 120 000, 140 000, 150 000, or a value in the range between any two of the aforementioned values, preferably between 10 000 and 100 000. Polyester polyurethane is the preferred polyurethane.

The molecular weight of the polycaprolactone may be 10 000, 20 000, 30 000, 40 000, 50 000, 60 000, 70 000, 80 000, 100 000, 200 000, 300 000, 400 000, 500 000, or a value in the range between any two of the aforementioned values, preferably between 10 000 and 60 000, more preferably between 37 000 and 500 000.

Caprolactone polyester polyurethane is particularly suitable, which polyurethane may be obtained by reacting isocyanate and polycaprolactone-based polyester. Such a caprolactone polyester polyurethane is commercially available as a granulate. The melting point of said polycaprolactone polyester polyurethane lies between 190 and 210 degrees Celsius. By adding the polycaprolactone, also preferably in granulate form, a thermoplastic composition is obtained that is distortable and kneadable at a temperature of - 69 degrees Celsius and remains distortable by cooling down about 50 degrees Celsius. At this temperature, the core layer **10** may be stretched at least up to twenty times the original length thereof. In the hardened condition, the thermoplastic composition is semi-rigid, unlike a typical rigid face mask, and has a memory effect that, after heating, returns to the shape formed on cooling. It is non-elastic in the hardened condition. The sheet **100** is able to secure the body part of the subject particularly when it is attached around the edges to a frame as described later below.

In an advantageous embodiment of the invention, the core layer **10** comprises between 1 to **40** % (w/w) of microspheres of non-metallic, heat-accumulating material which is especially suited for heating in a micro-wave oven. Preferred are glass microspheres with a diameter between 20 and 800 micrometer. A colouring agent may be added to the core.

### Intervening layers

One or more intervening layers **12, 12' (****FIG. 2****)** may be disposed between the core layer 10 and the first outer layer **5,** and/or disposed between the core layer **10** and the second outer layer **15.**

Typically an intervening layer **12, 12'** comprises a higher polycaprolactone content compared with the core layer **10,** and thus can be deformed at a lower temperature than the core layer **10.** According to one aspect of the invention, an intervening layer **12,** 12' has a polycaprolactone content that is higher than the core layer **10** by 5 %, 10 %, 15 %, 20 %, 25 %, 30 %, 50 %, 100 %, 200 %, 300 %, or 400 % or a value in the range between any two of the aforementioned values, preferably between 100 % and 400 %. An intervening layer **12, 12'** may be comprised of pure polycaprolactone.

Thickness of an intervening layer **12, 12'** may be 20%, 30%, 40%, 50%, 60%, 70% or 80% that of the core layer **10** or a value in the range between any two of the aforementioned values, preferably between 40 % and 60 %.

By virtue of an intervening layer **12, 12',** the sheet **100** can be deformed at a lower temperature, since only the only the intervening layer **12, 12'** of the sheet **100** needs to be heated above the temperature at which the thermoplastic material deforms; the result is that heating up time before use is shorter and comfort to the subject is improved. As the core layer **10** has not to be brought at such high temperature, the expansion of the core **10** is also smaller. This is especially advantageous when the sheet **100** is presented in the shape of a roll. Due to expansion when heating, successive windings could be pressed so strongly together than they adhere strongly to each other.

In an advantageous embodiment of the invention, the intervening layer **12, 12'** comprises between 1 to 40 % (w/w) of microspheres of non-metallic, heat-accumulating material which is especially suited for heating in a micro-wave oven. Preferred are glass microspheres with a diameter between 20 and 800 micrometer. A colouring agent may be added to the intervening layer **12, 12'.**

### First outer layer

The first outer layer **5,** also referred to as the 'first layer' herein, comprises a polyamide-based knitted fabric material with a thickness outside the core layer **10** of between 0.05 and **1.5** mm. According to one aspect of the invention, the knitted fabric material is formed from a yarn comprising between 80 % to 95 % polyamide, and between 5 % and 15 % elastane, preferably comprising 90 % polyamide and **10** % elastane. The fabric weight may be 190 g/m², 200 g/m², 210 g/m², 220 g/m², 230 g/m², or 240 g/m², preferably between 210 g/m² and 230 g/m². The fabric may be coloured, for example, with a calming colour such as a neutral tone, pastel shade, or primary colour such as blue.

The thickness of the first outer layer **5** is equal to or less than 0.05 mm, 0.06 mm, 0.08 mm, 0.1 mm, 0.5 mm, 0.8 mm, 1 mm, 1.2 mm, 1.5 mm or a value in the range between two of the aforementioned values, preferably between 0.05 and 1.5 mm, more preferably having a thickness of between 0.1 and 0.4 mm.

The first layer **5** is bonded to the core **10** or intermediate **12, 12'** layer by virtue of the adhesive property of the core **10** in the fluid condition. Adhesion may be enhanced pressing the first layer **5** onto the core layer **10,** for example, by the use of roller during manufacture.

Due to the presence of first layer **5,** the sheet **100** of the invention may be applied directly in distortable condition on the skin. It does not adhere to hair and skin. It does not either leave visible fingerprints, so that disposable gloves not necessary for the application. In addition, the first layer **5** forms a thin insulating layer, such that the skin is not subject to elevated temperatures that might otherwise harm such areas.

### Second outer layer

The second layer **15,** also referred to as the 'second layer' herein, comprises a soft resilient open cell foam plastic disposed over the core layer **10** and forming contact therewith or with the optional intermediate layer **12, 12'.**

The second layer **15** forms a physical and insulating layer which protects the core layer **10** in the malleable condition. The foam plastic outside the core layer **10** or optional intermediate layers **12, 12',** before the application, does not comprise thermoplastic material as for example the foam plastic from the composite material according to US-A 3,728,206 which foam plastic does not form a coating. The second layer **15** forms a covering which prevents portions of the sheet **100** from adhering inadvertently to one another. This allows the sheet to be transported in a deformable condition, rolled up, for example. The second layer **15** allows the sheet to be handled with bare hands without, damage to the core 10 or optional intermediate layers **12, 12',** and without adhering to the fingers.

The thickness of the second outer layer **15** is equal to or less than 0.05 mm, 0.06 mm, 0.08 mm, 0.1 mm, 0.5 mm, 0.8 mm, 1 mm, 1.2 mm, 1.5 mm or a value in the range between two of the aforementioned values, preferably between 0.05 and 1.5 mm, more preferably having a thickness of between 0.4 and 0.6 mm. It has been found that layers of foam plastic with a thickness outside of the core of more than 1.5 mm would prevent a good adherence to the underlying layer (i.e. core or intermediate layer) when the sheet is deformed or rolled up.

When the sheet is presented in the shape of a roll, the maximum thickness of the second layer **15** is preferably of 0.6 mm and when the material is used as a small strip for winding around body parts the thickness of the second layer **15** is preferably not higher than 0.4 mm.

The second layer **15** is made from a material able to withstand temperatures at which the core is softened or weakened. They are preferably made from a non-thermoplastic plastic, such as polyurethane formed into soft, open cell foam. The foam that has such an open-cell structure that core layer **10** in softened condition can traverse it when pressure is exerted but also that the second layer **15** can be elastically deformed without tearing. When the sheet **100** is heated, it can be stretched up to four times its original length without breakage of the second layer **15.** Suitable foam plastics for the second layer **15** are polyurethane, particularly polyester polyurethane and polyether foam.

The second layer **15** is bonded to the core **10** or intermediate **12, 12'** layer by virtue of the adhesive property of the core **10.** Adhesion may be enhanced pressing the second layer **15** onto the core layer **10,** for example, by the use of roller during manufacture.

For some applications, the second layer **15** may be provided with perforations (not shown) with a diameter of at least 0.5 mm and for example 2 mm. Such perforations assist, for example, with heating the core layer **10** by providing access directly to the core, and with drainage when heating the sheet occurs in a warm water bath.

Cross-wise through core **10,** first **5** and second **15** layers, smaller perforations with a diameter of at least 0.5 mm and preferably about 1 to 1.3 mm may be provided, so as not to hamper the skin breathing after applying the sheet. Said perforations may lie on rows crossing each other under 90 degrees and making an angle of **45** degrees with the transversal direction of the strip, plate or roll of composite material, at a distance of each other in the rows of 1.5 to 4 mm. The skin may still breathe even after application of the material.

### Attachment means

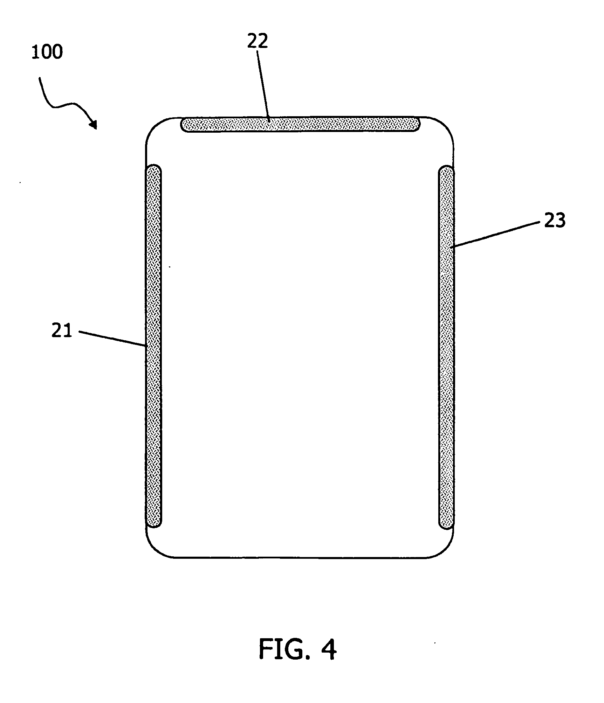

The sheet **100** for use with a frame **200** discussed below, may further comprise one or more attachment means **21, 22, 23 (****FIG. 4****),** configured to maintain the sheet **100** in position over the opening **56** provided by the frame **200.** The attachment means **21, 22, 23** is able to maintain the position of the sheet **100** when it supports the weight of the body portion (*e.g.* head) during treatment and when the sheet **100** receives the usual pressure applied to the head during its molding. The attachment means **21, 22, 23** preferably cooperates with a corresponding attachment means in the frame **200.** The attachment means **21, 22, 23** preferably is connected with at least two opposing edges of the sheet. According to one aspect of the invention, the attachment means **21, 22, 23** comprises a foldable edge, one part of a hook and loop fastener (*e.g.* Velcro strip), or one part of a zip fastener.

### Inflatable Cushion for use in medical treatment

One embodiment of the invention is an inflatable cushion **300** for use in medical treatments as describe above, at least partly made from the thermoplastic sheet **100** of the invention. The cushion **300** comprises a first **110** (*e.g.* upper) flexible and a second **115** (*e.g.* lower) flexible sheet that are sealed together, preferably around the edges so as form an inflatable cushion disposed with a lumen **117** in which inflation medium may be held. The first **110** and second **115** flexible sheets are impermeable to inflation medium. Where provided for the molding to and support of a body part, the cushion **300** is dimensioned to receive the body portion (*e.g.* the head) on the first sheet **115.**

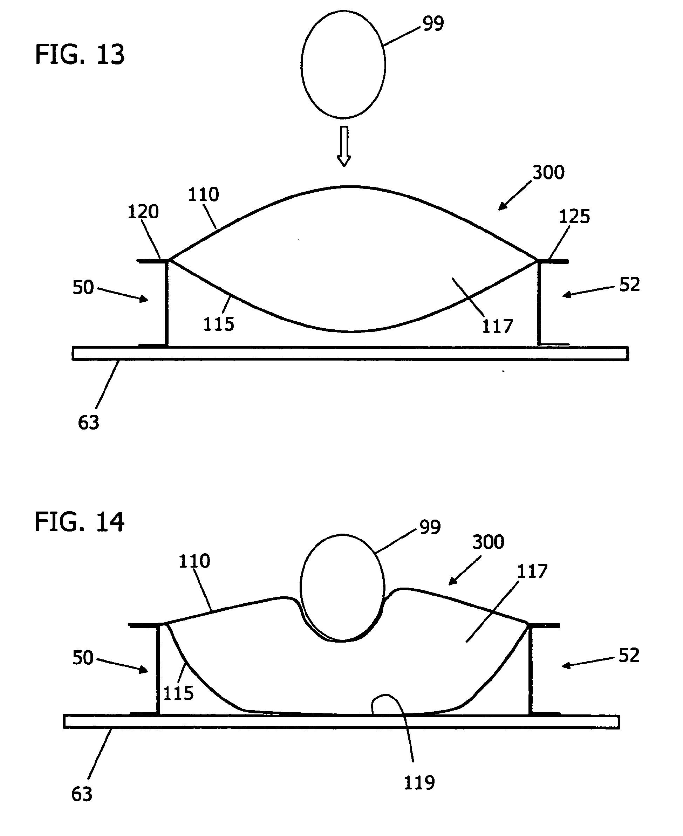

The first flexible surface **115** is at least partially, preferably entirely, formed from the thermoplastic sheet **100** of the invention, and thus is moldable in the heated and deformable condition to fit the contours of a body part applied thereto when the cushion **300** is in the inflated state. The first layer **5** of the thermoplastic sheet **100** faces outwards, while the second layer **15** faces the lumen **117** of the cushion **300.** Advantageously, inflation introduces a gap between the first flexible sheet **110** and the second flexible sheet **115** which allows the cushion **300** to be placed on a flattened surface such as a table during molding and prevents the first flexible sheet **110** from contacting the flattened surface while the body part is pressed onto the cushion, which would otherwise result in a false impression *e.g.* in a flattened plateau where both sheets **110, 115** touch the table, said impression not representative of the body part. Moreover, because of the medium inside, the cushion will automatically fit itself anatomically to the object, for example, to the back of the head and the neck. **FIGs. 13** and **14****,** showing transverse cross sections of a cushion 300 of the invention attached to table **63**-mounted longitudinal supports **50, 52** of a frame (described below) via seams **120, 125,** illustrate the sequence of taking an impression of a body part **99.** In **FIG. 13****,** the cushion **300** in a deformable (heated) condition is inflated, and the body part 99 applied thereto. To deform the cushion, it is typically heated in a water bath at **65** deg C. In **FIG. 14****,** the inflated cushion adapts to the shape of the body part. While the second sheet **115** of the cushion contacts the table **63** and forms a plateau **119,** the first sheet **110** remains true to the shape of the body part **99** being prevented from contacting the table **63** by virtue of the medium filled lumen 117. In other words, no false tension is obtained due to the fact that the part of the body **99** will not touch any additional support. During hardening, the pressure or volume of medium in the lumen can be adjusted in order to improve the shape of the impression. For example, medium can be added to or released from the lumen as the cushion cools. Once the first sheet **110** of the cushion has hardened, the cushion **300** may subsequently be used to immobilise the body portion in radiotherapy or other treatments. Hardening time for a cushion formed from sheet having a 2mm thickness of 2 mm is typically 3 minutes. After treatment, the cushion **300** may be remolded to fit another patient by reheating the thermoplastic sheet.

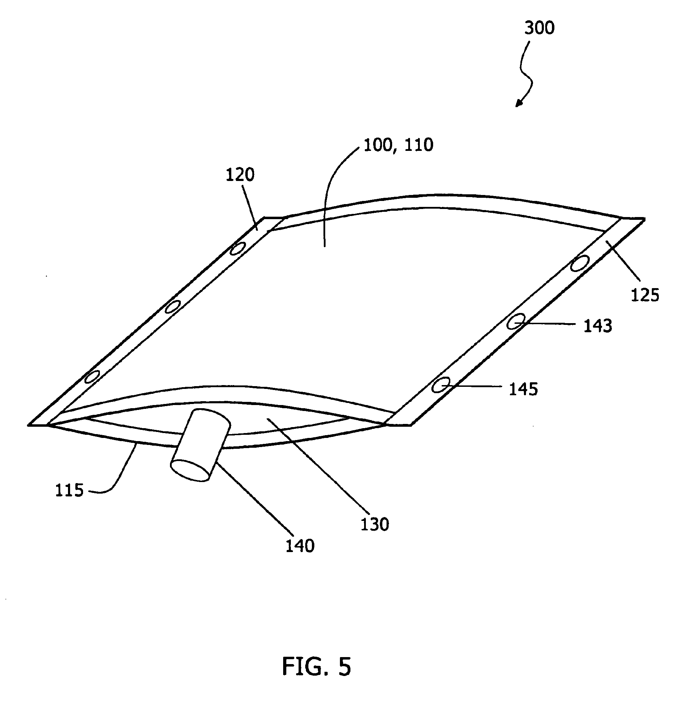

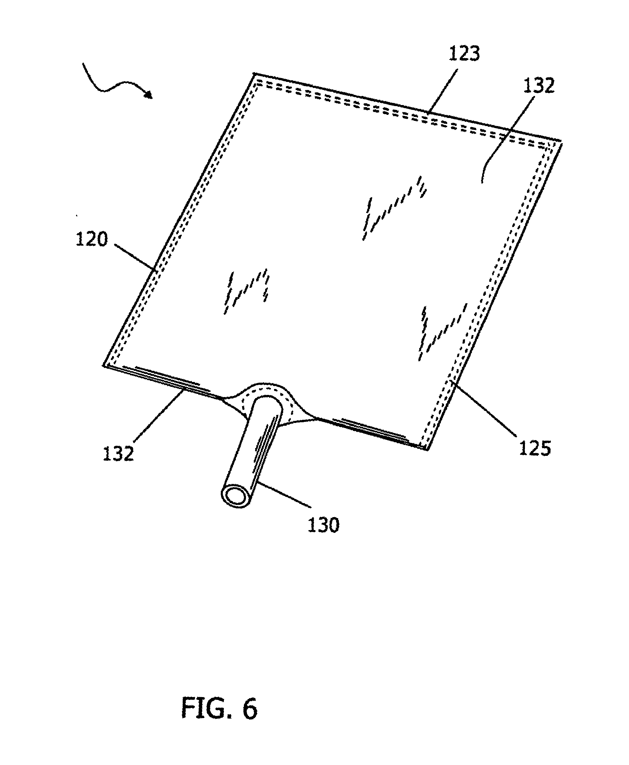

With reference to **FIGs. 5** and **6** inflatable cushion **300** of the invention comprises a first flexible sheet **110** formed from the thermoplastic sheet **100** of the invention, and a second flexible sheet **115** formed from a flexible material, that may be another thermoplastic sheet 100 of the invention or made from a heat resistive flexible material such as polyurethane. Preferably, the second flexible sheet **115** should not exhibit elastic properties. Both sheets, placed one over the other such that the second outer layers **15** are facing each other, and are sealed to form an inflation lumen **117,** typically by a sealing seam (*e.g.* **120, 125)** around the edges, for example, using adhesive, ultrasonic or heat sealing, or by the use of clamps. In a variation, shown in **FIG. 5** one or more side pleats **130** may be introduced between the first **110** and second **115** sheets that increase separation between the first **110** and second **115** flexible sheets after inflation. A side pleat **130** may be made from thermoplastic sheet **100** or from a heat resistive flexible material such as polyurethane. The nozzle is preferably attached using adhesive. **FIG. 5** depicts an instance of the invention having one side pleat **130** onto which the nozzle **140** is affixed.

In a preferred embodiment of the invention, and as shown in **FIG. 6** the cushion **300** is formed from a single thermoplastic sheet **100** of the invention, folded and sealed **(120, 123,125)** around the remaining three edges to form the inflation lumen **117.** The folded edge 132 is disposed with the inflation nozzle **140.**

The lumen **117** of the cushion **300** is in fluid connection with an inflation nozzle **140** configured to permit passage of an inflation medium which may be gaseous (*e.g.* air, oxygen, nitrogen) or liquid (*e.g.* saline or water). The inflation nozzle **140** may be disposed with a one way valve which prevents inflation medium from leaving the inflation lumen **117.** The valve may have a release mechanism that allows inflation medium to pass out from the lumen **117,** for example, by a pinching action. Alternatively, or in addition, the inflation nozzle **140** may be disposed with sealing cap. The inflation nozzle **140** is attached to the cushion **300** in a location that does not interfere with taking the impression, for example, at the edge of the cushion 300 as depicted in the figures. It should also be located so as to avoid interference with the attachment means (see below). In the case of a cushion for taking an impression of the head and neck, the nozzle is preferably located at the head end of the cushion.

The cushion **300** for use with a frame **200** discussed below, may further comprise one or more attachment means, configured to maintain the cushion 300 in position over an opening **56** provided by the frame **200.** The attachment means is configured to maintain the position of the cushion **300** when it supports the weight of the body portion (*e.g.* head) during treatment and when the cushion **300** receives the usual pressure applied to the body portion during molding. The attachment means cooperates with a corresponding attachment means in the frame, or co-operates with an intermediary buffering structure that connects the cushion attachment means with the frame attachment means. The attachment means is preferably disposed along two opposing edges of the cushion **300.** The attachment means may comprise one or more apertures **140, 143** located in a seam **125** of the cushion **300.** The attachment means may comprise opposing seams **120, 125** of the cushion **300;** which provide edges that can be clamped onto the frame.

### Buffering structure

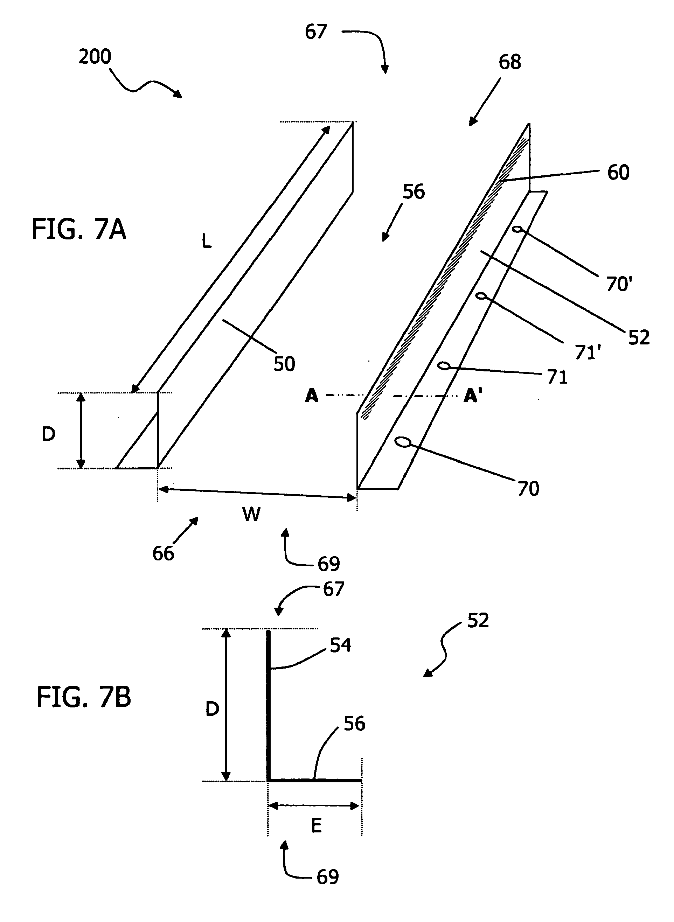

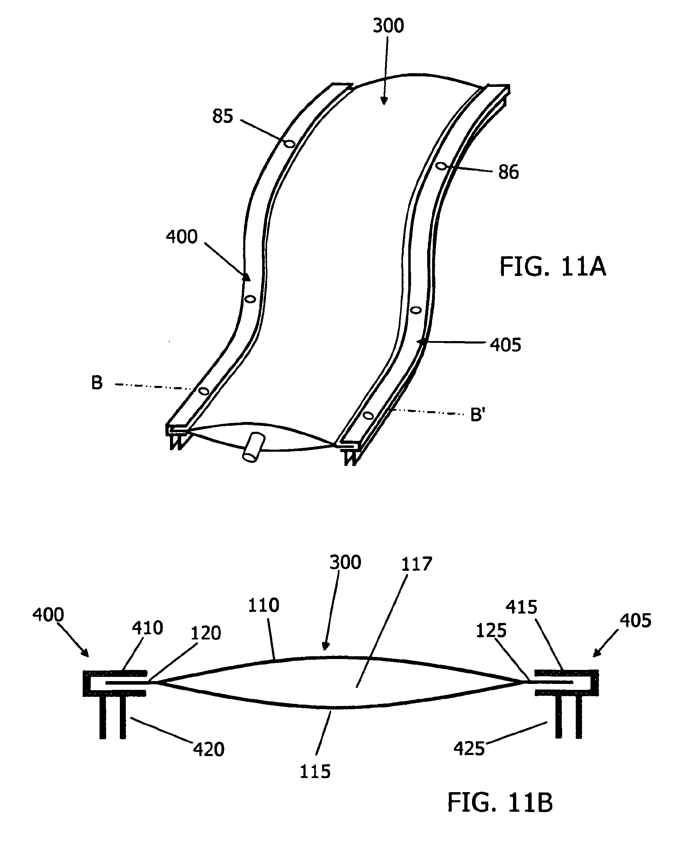

The buffering structure described herein is a structure that demountably attaches the cushion 300 to the frame 200 and maintains the cushion in a rigid position with respect to the frame. With reference to **FIGs. 11B****,** **15** and **16** the buffering structure **400, 405** comprises a cushion attachment element 410, 415, that receives and attaches the cushion 300, and a frame attachment element 420, 425 that receives and attaches the frame 200 preferably to the top of a long part 54 **(****FIG. 7B****)** of a longitudinal support **50, 52.** The cushion attachment element 410, 415 is rigidly fixed to the frame attachment element 420, 425 maintaining a constant juxtaposition between the frame 200 and cushion 300. A buffering structure 400, 405 is preferably formed from an elongate structure of rigid material, comprising cushion attachment element 410, 415 which is first elongated groove configured to receive the seam **120, 125** of a cushion 300. The frame attachment element 420, 425 according to one embodiment, is a second elongated groove configured to receive an edge of the frame 200 and the first groove is preferably perpendicular to the second groove as shown in FIG. **11B****.** The buffering structure may be provided with one or more apertures **85, 86** for insertion of securing pins which disengagably connect with the cushion seam.

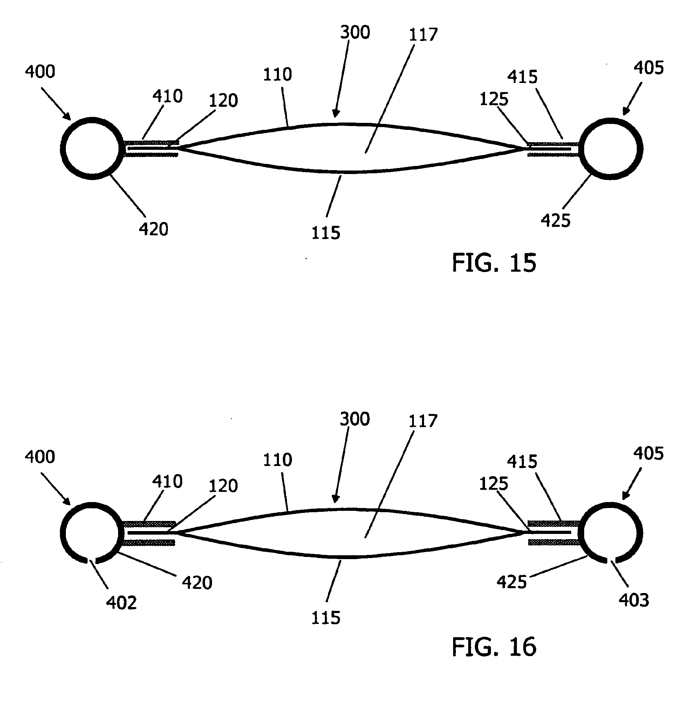

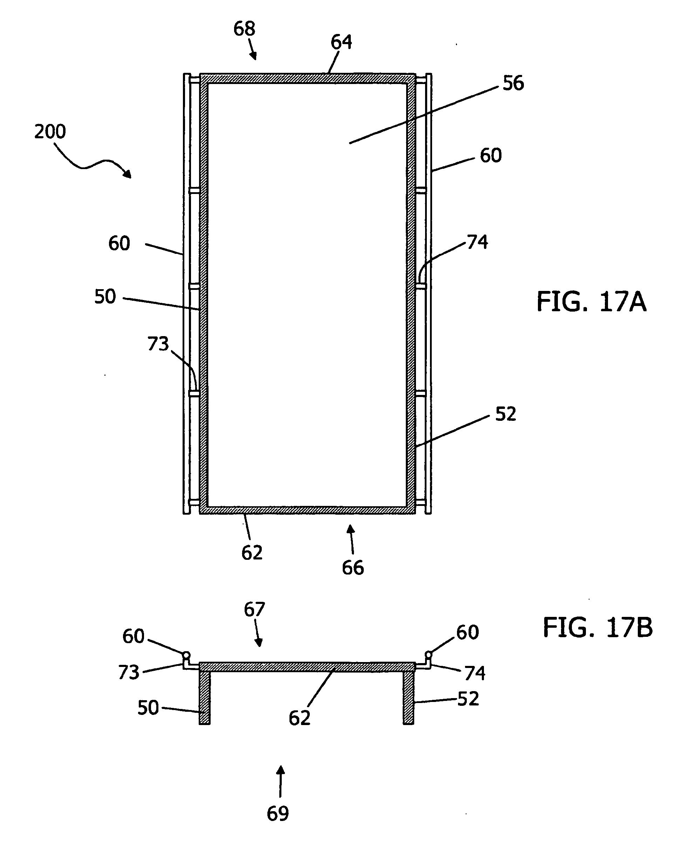

In an alternative embodiment shown in **FIG. 15****,** the frame attachment element 420, 425 is a hollow, longitudinal, tubular support, configured to receive an elongate cylindrical support rail extending the longitudinal length of the frame **200** (see, for example, **60.** **FIG. 17A 17B**). In variation shown in **PIG. 18,** the frame attachment element 420, 425 is a hollow, longitudinal, tubular support disposed with a longitudinal slit **402, 403**, configured to receive an elongate rail element extending the longitudinal length of the frame 200. The slit 402, 403 is configured to pass over any structural members (see, for example, **73.** 74, **FIG.1** **17A 17B****)** used to affix the rail to the frame.

### Moiding Frame

Further described herein is a device for molding a sheet 100 or cushion prior to medical treatment, and for immobilising a subject during medical treatment, especially radiotherapy to the head and neck, comprising a molding frame 200 that is configured to immobilise and support either a sheet of material 100 or the cushion 300 of the invention over an opening, which opening is dimensioned to receive the impression of a body part.

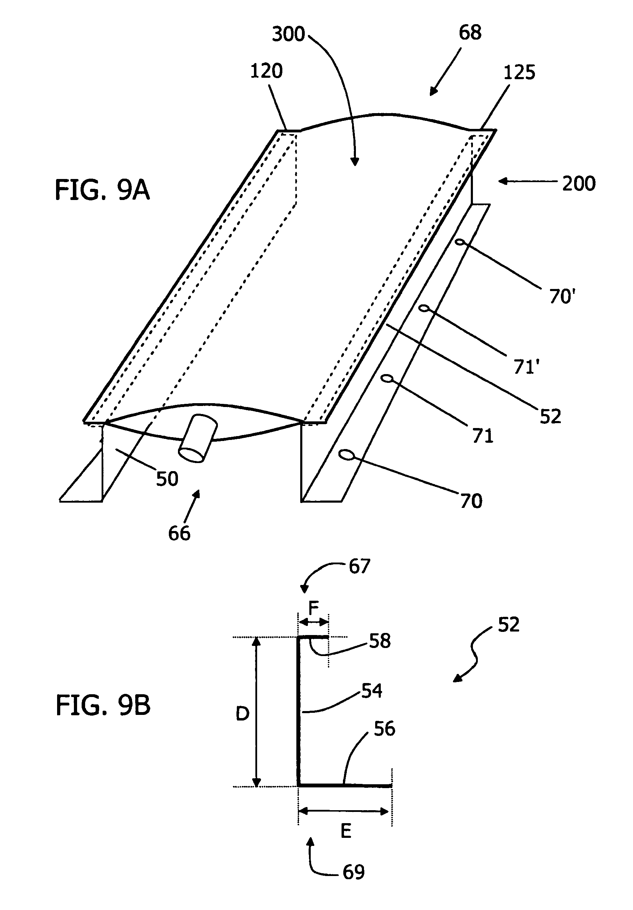

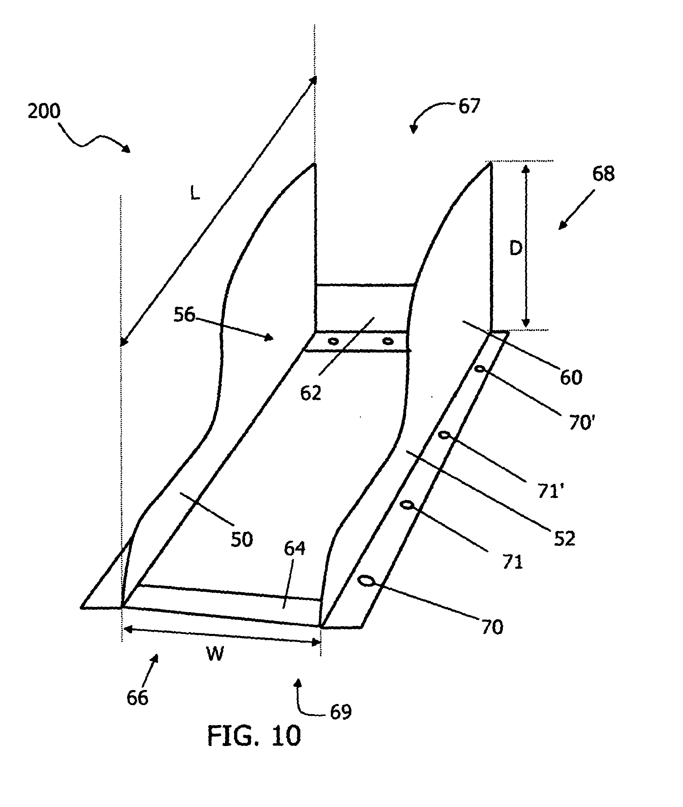

With reference to **FIGs. 7A** and **10****,** the molding frame **200** having a top end **67** and a base end **69,** comprises two rigid longitudinal supports **50, 52** arranged opposite each other, which flank an opening **56** configured to receive a sheet **100** or cushion **300** of the invention over the top **67** of the opening **56.** The frame **200** has an at least partially open first end **66** and an at least partially open second end **68** opposite thereto which allow access to the opening **56** when the top **67** of the frame is disposed with a sheet **100** or cushion **300.** As shown in **FIG. 9****,** the frame may be disposed with one or more cross-supports **62, 64** that rigidly link and separate the longitudinal supports **50, 52.** Where present, the cross supports will also flank the opening **56** *i.e.* be disposed at the ends **66, 68** of the frame. However, such cross-supports **62, 64** are not essential since the longitudinal supports **50, 52** may be separated by rigid attachment to the treatment table, to a rigid plate that couples to the treatment table, or to another rigidity providing means.

The supports **50, 52** comprise one or more securing means **70, 70'** at the base end adapted for demountable attachment to a treatment table or to a rigid plate that couples to the treatment table, or to other fixing means. The securing means **70, 70'** fixes the supports **50, 52** so they are immobilised relative to the table and to each other. The securing means 70, 70' may comprise one or more apertures that pass through the supports **50, 52.** The aperture can receive a bolt or pin that affixes the frame to treatment table or to a rigid plate provided with a reciprocating aperture. Once fixed, the supports **50, 52** cannot move without undue force, and are able to support the weight of the body part during treatment and also any usual pressure applied thereto during molding of the sheet **100.** After treatment, the supports 50, 52 can be demounted from the treatment table or from the rigid plate. The supports 50, 52 may further comprise additional securing means **71, 71'** that can receive and demountably secure a face mask. These additional securing means **71, 71'** may also be apertures that pass through the supports **50, 52.** Such additional aperture can receive a bolt or pin that affixes the face mask to the frame and optionally also to the treatment table or to a rigid plate.

The opening **56** formed by the frame **200** is dimensioned to receive the body portion (*e.g.* head) or more preferably, an impression thereof. The length, L, of the opening defined by the length of the longitudinal supports **50, 52,** and width, **W,** of the opening **56 (****FIGs. 7A** and 10) between the longitudinal supports **50, 52** will be determined in part by the dimensions of the treatment table, but general will be large enough to receive, for example, the head. The depth, **D,** between the top **67** and the base **69** of the opening **56** is sufficient to accommodate the body part, and to allow a gap between the deepest depression formed in the sheet **100** and the base of the aperture **56.**

Advantageously, the gap provides a route for excess water to drain from the sheet **100** after it has been retrieved from a water bath during deformation. The gap also provides a cooling channel for the circulation of air that assists rapid hardening.

It will be appreciated that the dimensions of the frame **200** will vary according to the body portion and on the size of the subject, however, as a general guidance, the dimensions of a frame to accommodate the shape of a head, may have a length, L. that is at most 25 cm, 30 cm, 35 cm, 40 cm, 45 cm, or 50 cm, or a value in the range between any two of the aforementioned values, preferably between 25 and 40 cm. The width **W,** may be at most 12 cm, 14 cm, 16 cm, 18 cm, 20 cm, 22 cm. 24 cm, 36 cm, 48 cm, 30 an, or a value in the range between any two of the aforementioned values, preferably between 12 and 24 cm. The depth D, may be at most 4 cm, 6 cm, 8 cm, 10 cm, 12 cm, 14 cm, 18 cm, or 18 cm, or a value in the range between any two of the aforementioned values, preferably between 4 and 10 cm.

In a preferred embodiment a longitudinal support **50, 52** comprises an L-shaped profile, the long part of the "L" providing depth, D, to the frame, and the narrow part of the L perpendicular, or adopting an angle between **85** deg and 95 deg to the long part and disposed with the aforementioned securing means **70, 70', 71, 71'**. **FIG. 6B** illustrates an L-shaped profile of a longitudinal support **50, 52** showing the long part **54** having depth, **D,** and perpendicular thereto the narrow part which extends from the base 69 of the long part **54** by an amount **E.** **FIG. 9B** also illustrates an L-shaped profile of a longitudinal support **50, 52** showing the long part **54** having depth, **D,** and perpendicular thereto the narrow part which extends from the long part **54** by an amount **E,** said longitudinal support **50, 52** further comprising a horizontal lip **58** which appears In the profile as an extension from the top **67** end of the long part **54.** The lip **58** extends from the long part **54** of the L-shaped profile by an amount **F.** As seen in **FIG. 9A****,** the lip **58** may provide an attachment means for a cushion of the invention.

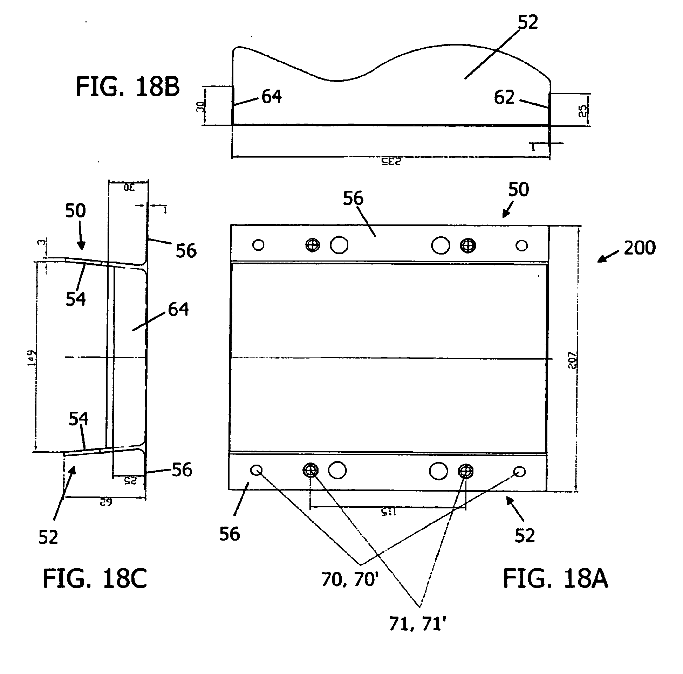

In the frame **200** depicted in **FIG. 7A****,** the depth D of the frame **200** may be essentially constant along the length of each support **50, 52.** depth **D** of the frame **200** may also vary along the length of each support **50, 52** which provides a side profile that better accommodates the shape of a body part **FIG. 10** shows an instance of a frame **200** whereby the depth **D** of the frame changes as a function of the length; in the illustrated embodiment, the frame is deeper at the second end **68** compared with the first end **66.** The depth between the first **66** and second **68** ends may vary In any manner, but preferably in a gradual fashion. The depth **D** frame may follow a path corresponding to a continuous curved line having only one point of inflection, which inflection point defines one convex part and one concave part. A particular embodiment of a frame is depicted in **FIGs. 18A to 18C** whereby the side profile is sinusoidal

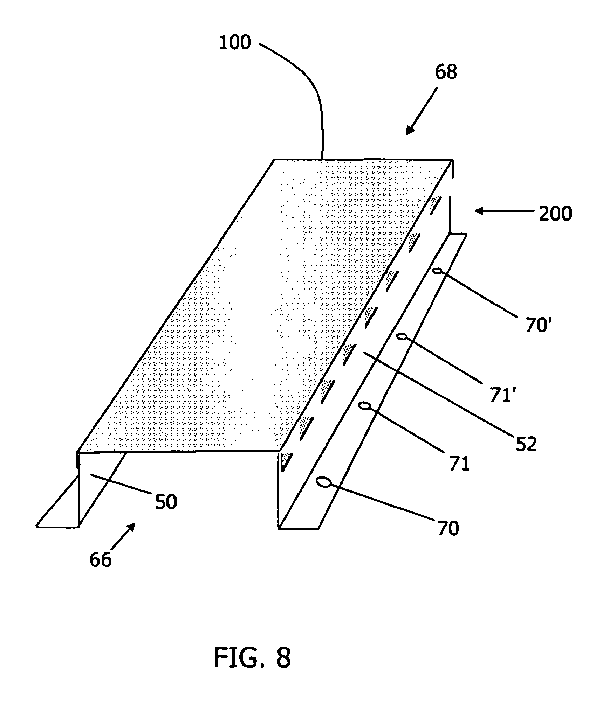

The supports **50, 52** further comprise one or more demountable attachment means **60** for the sheet **100** or cushion **300,** configured to maintain the sheet **100** or cushion **300** in position over the opening **56 as** shown In **FIGs. 8** and **9A** The attachment means **60** is able to maintain the position of the sheet **100** or cushion **300** when it supports the weight of the body portion (*e.g.* head) during treatment and when the sheet **100** or cushion 300 receives the usual pressure applied to the head during its molding. The attachment means **60** preferably cooperates with the corresponding edge of the sheet **100,** or with a seam edge of a cushion, or with a buffering structure **400, 405** attached to the cushion. The attachment means **60** is preferably disposed on the long part **54** of an L-shaped profile support **50, 52.** The attachment means **60** may comprise a spring clip, a screw-clip, one part of a hook and loop fastener (*e.g.* Velcro strip), or one part of a zip fastener. The attachment means may be comprised in a lip **58.** As mentioned elsewhere, the frame **200** may alternatively be attached to the cushion **300** via a buffering structure **400, 405.**

**FIGs 17A** and **17B** show a particular aspect whereby the frame **200** is dimensioned for taking an impression of the entire body ***i.e.*** resembles a bed frame. As with the other frames described herein, the bed frame **200** has a top end **67** and a base end **69,** comprises two rigid longitudinal supports **50, 52** arranged opposite each other, which flank an opening **56** configured to receive a sheet **100** or cushion **300** of the invention over the top **67** of the opening **56.** The frame **200** has an at least partially open first **end.66** and an at least partially open second end **68** opposite thereto which allow access to the opening **56** when the top **67** of the frame is disposed with a sheet 100 or cushion 300. The frame is disposed with cross-supports **62, 64** that rigidly link and separate the longitudinal supports **50, 52.** Each longitudinal support **50, 52** extends to the floor via one or more legs.

The opening **56** formed by the frame **200** is dimensioned to receive the whole body impression. The length of the opening defined by the length of the longitudinal supports **50, 52,** and width of the opening **56** between the longitudinal supports **50, 52** will be determined in part by the dimensions of the subject. The depth, between the top **67** and the base **69** of the opening **56** is sufficient to accommodate the body, and to allow a gap between the deepest depression formed in the sheet **100** and the base of the aperture **56.** Each supports **50, 52** further comprise a demountable attachment means **60,** that is an elongate rail for a receiving buffering structure **400, 405** as shown in **FIG. 16****,** which rail attaches to the longitudinal support **50, 52** using one or more bridging links **73, 74.** The cushion connected to two buffering structures as shown in **FIG. 16** attaches to the bed frame along rails.

### Inflatable Cushion for use as a container

Described herein is an inflatable cushion **300** at least partly made from the thermoplastic sheet **100** of the invention, wherein the first outer layer 5 is optional. Such cushion is suitable for non-therapeutic applications, for example, for a moldable medium container wherein the outer surface of the container does not need to provide comfort to the skin. Such container has application in the dead space of vehicles, sailing vessels etc. such as behind paneling, where they may be inflated and provide storage for media such as water and liquid fuel. Advantageously, dead space, normally difficult to access and having an irregular shape, can be utilised fully since the cushion expands to accommodate the otherwise unusable regions, regardless of the shape. The embodiments described above in regard of the construction of a cushion for medical treatments also apply to the cushion for use as an inflatable medium container. Most preferably, the cushion is formed from a single thermoplastic sheet 100 of the invention having an optional first outer layer, folded and sealed around the remaining three edges to form the inflation lumen. The folded edge is disposed with the inflation nozzle.

## Claims

1. A flexible thermoplastic sheet material (100), suitable for molding to and immobilising a part of a subject during medical treatment, comprising:
- a core layer (10) having an upper surface (1) and lower surface (2), that is a thermoplastic composition comprising polycaprolactone and polyurethane,
- a first outer layer (5) disposed over the upper surface of the core layer, comprising a material formed from a yam comprising polyamide and elastane,
- a second outer layer (15) disposed over the lower surface of the core layer comprising open cell foam
which layers are bonded so as to form a single sheet.

2. Sheet according to claim 1, wherein the core layer (10) comprises 20 % to 40 % polyurethane, and 60 % to 80 % (w/w) polycaprolactone.

3. Sheet according to claim 1, wherein the core layer (10) further comprises between 1 to 40 % (w/w) of non-metallic, heat-accumulating microspheres for heating in a microwave oven.

4. Sheet according to claim 1, wherein the yam of the first outer layer (5) comprises between 80 % to 95 % polyamide, and between 5 % and 15 % elastane.

5. Sheet according to claim 1, wherein the fabric weight of the first outer layer (5) is between 210 g/m² and 230 g/m².

6. Sheet according to claim 1, wherein the thickness of the first outer layer (5) is between 0.05 and 1.5 mm.

7. Sheet according to claim 1, wherein the second outer layer (15) is made from polyurethane, polyester polyurethane or polyether open-cell foam.

8. Sheet according to claim 1, further comprising an intervening layer **(12, 12')** disposed between the core layer (10) and the first outer layer (5), and/or disposed between the core layer (10) and the second outer layer (15), made from the same material as the core (10) and with a higher polycaprolactone content, or made from a material comprising pure polycaprolactone.

9. Sheet according to claim 1, having a maximum total thickness of 1.5 to **1.7** mm.

10. An inflatable cushion (300) dimensioned to receive a body portion comprising
- a first flexible sheet (110) made at least partially from a thermoplastic sheet (100) according to any of claims 1 to 9,
- a second flexible sheet (115), optionally made at least partially from a thermoplastic sheet according to any of claims 1 to 9.
which sheets are sealed together so as form a lumen (117) that can receive and retain inflation medium, and
- an inflation nozzle (140) for the passage of inflation medium to the lumen (117).

11. Inflatable cushion (300) according to claim 10, further comprising one or more side pleats (130) between the first (110) and second (115) flexible sheets.

12. Inflatable cushion according to claim 10 or 11, further comprising an attachment means **(120, 125, 143, 145)** for attachment of the cushion to a molding frame (300).

13. Inflatable cushion (300) according to any of claims 10 to 12, formed from a single piece of thermoplastic sheet (100) according to any of claims 1 to 9, folded and sealed so as form the lumen (117).

14. An inflatable cushion (300) comprising
- a first flexible sheet (110) made at least partially from a sheet (100) according to any of claims 1 to 9,
- a second flexible sheet (115), optionally made at least partially from a sheet according to any of claims 1 to 9,
which sheets are sealed together so as form a lumen (117) that can receive and retain inflation medium, and
- an inflation nozzle (140) for the passage of inflation medium to the lumen (117).

15. Inflatable cushion (300) according to 14 claim, further comprising one or more side pleats (130) between the first (110) and second (115) flexible sheets.

16. Inflatable cushion (300) according to claim 14 or 15, formed from a single piece of thermoplastic sheet (100) according to any of claims 1 to 9, folded and sealed so as form the lumen **(117).**

## Patentansprüche

1. Ein flexibles thermoplastisches Folienmaterial (100), geeignet für Guß zu und Immobilisierung Teils eines Subjekts während einer medizinischen Behandlung, umfassend:
- eine Kernschicht (10) mit einer oberen Oberfläche (1) und einer unteren Oberfläche (2), die eine thermoplastische Zusammenstellung ist, umfassend Polycaprolactone und Polyurethan,
- einer ersten Außenlage (5) angeordnet über der oberen Oberfläche der Kernschicht, umfassend einem Material, aus einem Garn geformt, umfassend Polyamid und Elastan,
- einer zweiten Außenlage (15), angeordnet über der unteren Oberfläche der Kernschicht umfassend offenporiges Schaum,
wobei die Lagen verbunden sind so dass sie ein einzelnes Blatt formen.

2. Folienmaterial nach Anspruch 1, wobei die Kernschicht (10) umfasst 20% bis 40% Polyurethan, und 60% bis 80% (w/w) Polycaprolactone.

3. Folienmaterial nach Anspruch 1, wobei die Kernschicht (10) weiterhin zwischen 1% bis 40% (w/w) nicht-metallische, wärmespeichernde Mikrosphären zur Erwärmung in der Mikrowelle umfasst.

4. Folienmaterial nach Anspruch 1, wobei das Garn der ersten Außenlage (5) zwischen 80% bis 95% Polyamid, und zwischen 5% und 15% Elastan umfasst.

5. Folienmaterial nach Anspruch 1, wobei das Stoffgewicht der ersten Außenlage (5) zwischen 210 g/m² und 230 g/m² liegt.

6. Folienmaterial nach Anspruch 1, wobei die Dicke der ersten Außenlage (5) zwischen 0,05 und 1,5 mm liegt.

7. Folienmaterial nach Anspruch 1, wobei die zweite Außenlage (15) hergestellt ist aus Polyurethan, Polyester-Polyurethan oder Polyether offenporigen Schaum.

8. Folienmaterial nach Anspruch 1, weiterhin umfassend eine Zwischenlage (12, 12'), angeordnet zwischen der Kernschicht (10) und der ersten Außenlage (5), und/oder angeordnet zwischen der Kernschicht (10) und der zweiten Außenlage (15), hergestellt vom demselben Material als die Kernschicht (10), und mit einem höheren Polycaprolactone-Gehalt, oder hergestellt aus einem Material umfassend reines Polycaprolactone.

9. Folienmaterial nach Anspruch 1, die eine maximale Gesamtdicke von 1,5 bis 1,7 mm aufweist.

10. Ein aufblasbares Kissen (300) dimensioniert zur Aufnahme eine Portion von einem Körper umfassend
- eine erste flexible Folie (110) zumindest teilweise hergestellt aus dem thermoplastischen Folienmaterial (100) gemäß einem der Ansprüche 1 bis 9;
- eine zweite flexible Folie (115) optional zumindest teilweise hergestellt aus dem thermoplastischen Folienmaterial (100) gemäß einem der Ansprüche 1 bis 9;
welche Folienmaterialen zusammen versiegelt sind, so dass sie einen Lumen (117) bilden, was ein Aufblasmedium empfangen und behalten kann, und
- eine Inflation Düse (140) für die Durchleitung des Aufblasmediums zum Lumen (117).

11. Aufblasbares Kissen (300) nach Anspruch 10, weiter umfassend ein oder mehrere Seitenfalten (130) zwischen dem ersten (110) und dem zweiten (115) flexiblen Folienmaterialien.

12. Aufblasbares Kissen nach Anspruch 10 oder 11, weiterhin umfassend Befestigungsmittel (120, 125, 143, 145) zum Befestigung des Kissens an einem Guß-Rahmen (300).

13. Aufblasbares Kissen (300) nach einem der Ansprüche 10 bis 12, so gebildet aus einem einzelnen Stück der thermoplastischen Folie nach einem der Ansprüche 1 bis 9, gefaltet und versiegelt dass sie das Lumen (117) bilden.

14. Ein aufblasbares Kissen (300) umfassend
- eine erste flexible Folie (110) zumindest teilweise hergestellt aus dem Folienmaterial (100) nach einem der Ansprüche 1 bis 9,
- eine zweite flexible Folie (115), optional zumindest teilweise hergestellt aus dem Folienmaterial nach einem der Ansprüche 1 bis 9,
welche Folienmaterialien so versiegelt sind das einen Lumen (117) gebildet wird das Aufblasmedium empfangen und behalten kann, und
- eine Inflation Düse (140) für die Durchleitung des Aufblasmediums zum Lumen (117).

15. Aufblasbares Kissen (300) nach Anspruch 14, weiter umfassend eine oder mehrere Seitenfalten (130) zwischen dem ersten (110) und dem zweiten (115) flexible Folien.

16. Aufblasbares Kissen (300) nach einem der Ansprüche 14 oder 15, gebildet aus einem einzelnen Stück der thermoplastischen Folienmaterial (100) nach einem der Ansprüche 1 bis 9, gefaltet und versiegelt, so wie sie das Lumen (117) bilden.

## Revendications

1. Une feuille flexible en matériau thermoplastique (100) adaptée au moulage et à l'immobilisation d'une partie d'un sujet au cours d'un traitement médical, comprenant :
- Une couche de base (10) ayant une surface supérieure (1) et une surface inférieure (2), qui est de composition thermoplastique comprenant du polycaprolactone et du polyuréthane,
- une première couche externe (5) disposée sur la surface supérieure de la couche de base, comprenant un matériau constitué à partir d'un filé comprenant du polyamide et de l'élasthanne,
- une seconde couche externe (15) disposée sur la face inférieure de la couche de base comprenant de la mousse à cellules ouvertes,
lesquelles les couches sont collées pour former une seule feuille.

2. Feuille, selon la revendication 1, dans laquelle la couche de base (10) comprends 20% à 40% de polyuréthane et 60% à 80% (w/w) de polycaprolactone.

3. Feuille, selon la revendication 1, dans laquelle la couche de base (10) comprend en outre entre 1 et 40% (w/w) de microsphères non-métalliques, accumulateurs de chaleur pour chauffage dans un four à micro-onde.

4. Feuille, selon la revendication 1, dans laquelle le filé de la première couche externe (5) comprend entre 80% et 95% de polyamide et entre 5% et 15% d'élasthanne.

5. Feuille, selon la revendication 1, dans laquelle le poids du tissu de la première couche externe (5) est entre 210 g/m² et 230g/m².

6. Feuille, selon la revendication 1, dans laquelle l'épaisseur de la première couche externe (5) est entre 0.05 et 1.5 mm.

7. Feuille, selon la revendication 1, dans laquelle la seconde couche externe (15) est fabriquée de polyuréthane, polyuréthane polyester ou de mousse de polyéther à cellules ouvertes.

8. Feuille, selon la revendication 1, comprenant en outre une couche intervenante (12, 12') disposée entre la couche de base (10) et la première couche externe (5) et/ou disposée entre la couche de base (10) et la seconde couche externe (15), fabriquée du même matériau que la couche de base (10) et avec une teneur plus élevée en polycaprolactone ou fabriquée d'un matériau comprenant du polycaprolactone pure.

9. Feuille, selon la revendication 1, ayant une épaisseur totale maximale de 1.5 à 1.7 mm.

10. Un coussin gonflable (300) dimensionné pour recevoir une partie du corps comprenant
- une première feuille flexible (110) au moins partiellement fabriquée à partir d'une feuille thermoplastique (100) selon une quelconque des revendications 1 à 9,
- une seconde feuille flexible (115), facultativement fabriquée au moins partiellement d'une feuille thermoplastique selon une quelconque des revendications 1 à 9,
lesquelles les feuilles sont soudées ensemble pour former un lumen (117) pouvant recevoir et retenir un milieu de gonflage, et
- une buse de gonflage (140) pour le passage du milieu de gonflage vers le lumen (117).

11. Coussin gonflable (300), selon la revendication 10, comportant en outre un ou plusieurs plis latéraux (130) entre la première (110) et la seconde (115) feuille flexible.

12. Coussin gonflable selon les revendications 10 ou 11, comprenant en outre un moyen de d'attachement (120, 125, 143, 145) pour l'attachement du coussin à un châssis de moulage (300).

13. Coussin gonflable (300) selon l'une quelconque des revendications 10 à 12, fabriqué d'une seule pièce de feuille thermoplastique (100) selon l'une quelconque des revendications 1 à 9, plié et scellé de manière à former le lumen (117).

14. Un coussin gonflable (300) comprenant
- une première feuille flexible (110) fabriquée au moins partiellement à partir d'une feuille (100) selon l'une quelconque des revendications 1 à 9,
- une seconde feuille flexible (115) éventuellement fabriquée au moins partiellement à partir d'une feuille selon l'une quelconque des revendications 1 à 9, lesquelles les feuilles sont soudées ensemble pour former un lumen (117) pouvant recevoir et retenir le milieu d'inflation, et
- une buse de gonflage (140) pour le passage du milieu de gonflage vers le lumen (117).

15. Coussin gonflable (300) selon la revendication 14, comprenant en outre un ou plusieurs plis latéraux entre la première (110) et la seconde (115) feuille flexible.

16. Coussin gonflable (300) selon les revendications 14 ou 15, formé d'une seule pièce de feuille thermoplastique (100) selon l'une quelconque des revendications 1 à 9, plié et scellé de manière à former le lumen (117).
